# EUROPÄISCHE PATENTANMELDUNG

(11) **EP 3 165 216 A1**
(43) Veröffentlichungstag der Anmeldung: **10.05.2017**
(21) Anmeldenummer: 15193101.1
(22) Anmeldetag: 05.11.2015
(51) Int. Cl.: A61K 8/34, A61K 8/37, A61Q 19/00, A61K 8/81, A61K 8/06, A61K 8/02

(54) **TEXTURIERTE ZUSAMMENSETZUNG**

(71) Anmelder: BASF SE, 67056 Ludwigshafen am Rhein (DE)
(72) Erfinder: Riedel, Heidi, 40545 Duesseldorf (DE); Klotz, Bjoern, 40699 Erkrath (DE); Jung, Robert, 40789 Monheim (DE)
(74) Vertreter: Blumberg vel Spalve, Jutta

(57) **Zusammenfassung**

Die vorliegende Erfindung beschreibt Zusammensetzungen, die in Öl-in-Wasser-Systemen und wässrigen Systemen bestimmte Texturen bilden und insbesondere bei der Herstellung von kosmetischen Produkten Anwendung finden.

## Beschreibung

### 1. Gebiet der Erfindung

Die vorliegende Erfindung betrifft texturierte Zusammensetzungen insbesondere für die Kosmetikindustrie, die einem Öl-in-Wasser System oder in einem wässrigen System bestimmte molekulare Strukturen bilden. Diese Zusammensetzungen bilden interessante Texturen, die beispielsweise wie "Crushed Ice", oder wie eine "Mousse" aussehen.

### 2. Hintergrund der Erfindung

Ein klarer Blick für die Bedürfnisse der Verbraucher und das Erkennen zukünftiger Trends sind entscheidend für die Entwicklung erfolgreicher Körperpflegeprodukte. Mit maßgeschneiderten Produkten und Lösungen für die Kosmetik sollen die Chancen und Möglichkeiten ausgeschöpft werden, die in diesen neuen Trends liegen.

Zu diesen neuen Trends gehört auch die Entwicklung von Texturen von Kosmetikprodukten. Texturen beeinflussen den Trage- und Applikationskomfort von Kosmetikprodukten und vermitteln dem Verbraucher einen Eindruck hinsichtlich der Sensorik des Produktes.

EP 1 212 042 B1 beschreibt eine Strukturierungszusammensetzung, die in einem Öl-in-Wasser System ein zweischichtiges lamellares Gelnetzwerk bildet. Diese Zusammensetzung enthält ein kationisches Quellmittel, bei dem es sich um ein Fettsäureaddukt von Amidopropyldimethyl-2-hydroxyethylammoniumhalogenid handelt und eine Geliermittelmischung von Emulgatoren mit niedrigem HLB-Wert, die aus Fettalkoholen und - estern ausgewählt sind. Das Verhältnis zum anionischen Emulgator zum Fettalkohol ist äußerst gering angesetzt. Diese Produktstrukturierungszusammensetzungen können in Körperpflegeformulierungen zur Strukturbildung verwendet werden.

Kosmetische Öl-in-Wasser Emulsionen basieren üblicherweise auf einer Mikrostruktur aus feinstverteilten Öltröpfchen in einer äußeren Wasserphase. Diese Emulsionen zeichnen sich durch eine weiße homogene Textur aus. Es besteht allerdings verstärkt ein Bedarf nach neuartigen Erlebnissen während der Anwendung der Pflegeemulsionen.

Die Aufgabe der vorliegenden Erfindung besteht darin, außergewöhnliche Texturen zur Verfügung zu stellen, die sich optisch von den homogenen weißen Öl-in-Wasser Emulsionen abheben und dem Verbraucher bei der Anwendung des Produkts auf der Haut neuartige sensorische Erlebnisse bieten.

### 3. Zusammenfassung der Erfindung

Die vorliegende Erfindung betrifft eine texturierte Zusammensetzung, die in einem Öl-in-Wasser-System ein lamellares Gelnetzwerk bildet und die Bestandteile aufweist:
- Eine Emulgatorkombination aus
   0,05 bis 3,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- 3,00 bis 20,0 Gew.-% mindestens ein Öl und/oder Wachs, und
- bis 100 Gew.-% Wasser,
wobei das Verhältnis von anionischem Emulgator zu Fettalkohol im Bereich von 1:6 bis 2:3 liegt. Diese Emulsion zeigt eine durchscheinende, glasige, "bruchartige" Optik, die ihrem Aussehen nach "Crushed Ice" ähnlich sieht.

Die Erfindung betrifft weiterhin eine texturierte Zusammensetzung, die in einem wässrigen System ein Hydrogel bildet und die Bestandteile aufweist:
- Eine Emulgatorkombination aus
   0,05 bis 3,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- bis 100 Gew.-% Wasser,
wobei das Verhältnis von anionischem Emulgator zu Fettalkohol im Bereich von 1:3 beträgt. Die Zusammensetzung weist die Textur eines Peelinggels auf, wobei kristalline Partikel in dem klaren Gel ausgebildet sind.

Die Erfindung betrifft weiterhin eine texturierte Zusammensetzung, die in einem Öl-in-Wasser-System eine schaumartige Struktur bildet und die Bestandteile aufweist:
- Eine Emulgatorkombination aus
   0,05 bis 3,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- 3,00 bis 20,0 Gew.-% mindestens ein Öl und/oder Wachs, und
- bis 100 Gew.-% Wasser,
wobei die Emulgatorkombination 3,5 bis 5,5 Gew.-% beträgt und das Verhältnis von anionischem Emulgator zu Fettalkohol im Bereich von 6:1 bis 3:2 liegt. Diese Zusammensetzung liegt in Form einer Mousse vor, mit einer schaumförmigen, soufleeartigen Textur, die Luftbläschen aufweist.

Schließlich betrifft die vorliegende Erfindung eine texturierte Zusammensetzung, in einem Öl-in-Wasser-System eine feindisperse Tröpfchenverteilung mit lamellaren Strukturen bildet und die Bestandteile aufweist:
- Eine Emulgatorkombination aus
   0,05 bis 5,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- 0,10 bis 20,0 Gew.-% mindestens ein Öl und/oder Wachs, und
- bis 100 Gew.-% Wasser,
wobei die Emulgatorkombination 2,00 bis 8,00 Gew.-% beträgt und das Verhältnis von anionischem Emulgator zu Fettalkohol 1:1 beträgt. Die Zusammensetzung liegt in Form einer Emulsion mit cremeartiger Textur vor.

Alle Mengenangaben beziehen sich auf 100 % (Gesamtmenge) der texturierten Zusammensetzung.

Alle texturierten Zusammensetzungen zeichnen sich durch eine hervorragende Lagerstabilität über einen Zeitraum bis zu 3 Jahren aus. Auch bei höheren Temperaturen (>40°C) oder Wechseltemperaturen (-5°C bis 40°C) sowie Gefriertemperaturen wird eine Lagerstabilität bis zu 6 Monaten erreicht.

Die vorliegende Erfindung betrifft auch Verfahren zur Herstellung dieser texturierten Zusammensetzungen sowie texturierte kosmetische Zusammensetzungen.

Die lamellare Gelnetzwerkstruktur der "Crushed Ice"-Textur ist in den Figuren 1 bis 4 anschaulich gezeigt. Es zeigen:
- Figur 1: eine elektronenmikroskopische Aufnahme einer erfindungsgemäßen ÖI-in-Wasser-Emulsion in der Vergrößerung 1500 :1;
- Figur 2: eine elektronenmikroskopische Aufnahme einer erfindungsgemäßen Öl-in-Wasser-Emulsion in der Vergrößerung 5000 : 1;
- Figur 3: eine elektronenmikroskopische Aufnahme einer erfindungsgemäßen Öl-in-Wasser-Emulsion in der Vergrößerung 15000 : 1; und
- Figur 4: eine elektronenmikroskopische Aufnahme einer erfindungsgemäßen Öl-in-Wasser-Emulsion in der Vergrößerung 15000 : 1

### 4. Beschreibung der Erfindung

Die vorliegende Erfindung beschreibt Zusammensetzungen, die in einem Öl-in-Wasser-System ein lamellares Gelnetzwerk bilden. Die erhaltene Textur weist eine durchscheinende, glasige, bruchartige Optik, analog Crushed Ice, auf. Diese Zusammensetzungen sind durch die lamellare Gelnetzwerkstruktur stabilisiert, was im Gegensatz zur klassischen Emulsionsstruktur mit fein verteilten Öltröpfchen in der äußeren Wasserphase steht. Die Zusammensetzungen sind nicht homogenisiert, sondern unter moderaten Bedingungen gerührt und zeichnen sich durch eine ausgezeichnete Langzeitstabilität aus.

Die erfindungsgemäßen Zusammensetzungen enthalten eine Emulgatorkombination aus 0,05 bis 3,0 Gew.-% mindestens einem anionischen Emulgator und 0,15 bis 9,00 Gew.-% mindestens einem Fettalkohol.

Unter Fettalkoholen sind primäre aliphatische Alkohole der Formel (I) zu verstehen,

R-OH (I)

worin R ein aliphatischer, linearer oder verzweigter Alkylrest mit 6 bis 22 C-Atomen, bevorzugt 14 bis 20 C-Atomen, mit 0 und/oder 1, 2 oder 3 Doppelbindungen bedeutet.

Bevorzugt ist der Fettalkohol in einer Konzentration von 0,5 bis 6,00 Gew.-% in der Zusammensetzung enthalten.

Typische Beispiele sind Capronalkohol, Caprylalkohol, 2-Ethylhexylalkohol, Caprinalkohol, Laurylalkohol, Isotridecylalkohol, Myristylalkohol (Lanette® 14), Lanette® 14/MB), Cetylalkohol (Lanette® 16), Palmoleylalkohol, Stearylalkohol (Lanette® 18), Cetearylalkohol (Lanette® D), Isostearylalkohol, Oleylalkohol, Elaidylalkohol, Petroselylalkohol, Linolylalkohol, Linolenylalkohol, Elaeostearylalkohol, Arachylalkohol, Gadoleylalkohol, Behenylalkohol (Lanette® 22), Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol. Ganz besonders bevorzugte Fettalkohole sind Cetylalkohol, Stearylalkohol und Cetearylalkohol.

Das in der texturierten Zusammensetzung enthaltende Hydrokolloid ist aus der Gruppe Acrylsäure und Acrylsäurederivate, Kohlenhydrate, wie Cellulose und natürliche Gummi und deren Derivate gewählt. Bevorzugte Hydrokolloide sind Polyacrylate (Cosmedia® SP und Cosmedia® ACE, BASF), Carbomer (Rheocare® C Plus, BASF), Acrylat-Copolymer (Rheocare® TTA). Ein besonders bevorzugtes Hydrokolloid ist ein Carbomer.

Die Konzentration des Hydrokolloids in der Zusammensetzung liegt bevorzugter Weise in einem Bereich von 0,1 bis 1,00 Gew.-%.

Der anionische Emulgator in der texturierten Zusammensetzung der vorliegenden Erfindung kann in der Regel jeder anionische Emulgator sein, der für den angestrebten Zweck geeignet ist.

Der anionische Emulgator ist bevorzugter Weise mindestens ein Emulgator aus der Gruppe Salze der Fettsäuren, Salze der Fettsäuren in Kombination mit Mono-/Diglyceriden der Fettsäuren, Salze der Stearoylmilchsäure, Salze der Stearoylglutaminsäure bzw. Alkylglutamate, Alkylphosphate, Alkylsulfate, Alkylsarkosinate, Salze der Alkylsulfobernsteinsäure und Salze der Zitronensäureester. Bevorzugte anionische Emulgatoren sind Glycerylstearat/Stearinsäure (Cutina® FS 45, BASF), Natriumstearoylglutamate (Eumulgin® SG, BASF), Dinatrium-Cetearylsulfosuccinat (Eumulgin® Prisma, BASF), Natriumcetearylsulfate (Lanette® E, Lanette® N, Lanette® SX BASF). Besonders bevorzugte anionische Emulgatoren sind Glycerylstearat/Stearinsäure, Natriumstearoylglutamat und Dinatrium-Cetearylsulfosuccinat.

In einer bevorzugten Ausführungsform besteht der anionische Emulgator aus einer Mischung aus Fettsäuren und Mono-/Diglyceriden dieser Fettsäuren im Verhältnis von 1:99 bis 20:80. Die Gesamtkonzentration beträgt bevorzugt 0,25 bis 3 Gew.-%, bevorzugt 0,5 bis 2 Gew.-%, bezogen auf die Zusammensetzung.

Das Öl und/oder Wachs in der erfindungsgemäßen texturierten Zusammensetzung ist aus der Gruppe Fettsäureester, Kohlenwasserstoffe, Guerbetalkohole, Tri- oder Partialglyceride, Mono-/Dialkylether, Mono-/Dialkylcarbonate, öllösliche UV-Filter, Fettalkoholether, mikrokristalline Wachse, Mineralöl, Silikonöl, natürliche pflanzliche Öle und deren Mischungen ausgewählt. Die Konzentration des Öls in der Zusammensetzung beträgt bevorzugt 5,00 bis 15,00 Gew.-%.

Bevorzugte Öle und/oder Wachse sind Guerbetalkohole auf Basis von Fettalkoholen mit 6 bis 18, vorzugsweise 8 bis 10 Kohlenstoffatome (Eutanol G, Eutanol G 16), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat (Cetiol MM), Myristylpalmitat, Myristylstearat, Myristylisostearat, Myristyloleat, Myristylbehenat, Myristylerucat, Cetylmyristat, Cetylpalmitat (Cutina CP), Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Isopropyl Myristate, Isopropyl Palmitate, Oleylmyristat, Oleylpalmitat, Oleylstearat, Oleylisostearat, Oleyloleat, Oleylbehenat, Oleylerucat (Cetiol J 600), Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Ethylhexyl Stearate (Cetiol 868), Hexyl Laurate (Cetiol A), C12.15 Alkyl Benzoate (Cetiol AB), Dibutyl Adipate (Cetiol B), Coco-Caprylate (Cetiol C5), Coco-Caprylate/Caprate (Cetiol LC, Cetiol C 5C), Propylheptyl Caprylate (Cetiol Sensoft), Cetearyl Isononanoate (Cetiol SN), Decyl Oleate (Cetiol V), Cetearyl Ethylhexanoate (Luvitol EHO), Daneben eignen sich Ester von C₁₈-C₃₈-Alkylhy-droxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) wie Propylene Glycol Dicaprylate/Dicaparte (Myritol PGDC). und/oder Guerbetalkoholen, Triglyceride auf Basis C₆-C₁₀-Fettsäuren flüssige Mono-/Di-/Triglyceridmischungen auf Basis von C₆-C₁₈-Fettsäuren (Myritol 331, Myritol 312, Myritol 318), , Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN, Cetiol AB), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Cetiol E). Weitere geeignete Emollients sind pflanzliche Öle(Cegesoft GPO, Cegesoft PFO, Cegesoft PS 6, Cegesoft SBE, Cegesoft SH) und Mischungen daraus (Cegesoft VP), Silikonöle, Kohlenwasserstoffe wie Cetiol Ultimate, Hydrogenated Polyisobutene (Luvitol Light), Mineralöle, Isoparaffine, Paraffine.

Besonders bevorzugte Öle sind hoch- bzw. mittelpolare Öle, wie bespielsweise Coco-Caprylat/Caprat (Cetiol® LC), Ethylhexyl Palmitat (CEGESOFT^{®} C24), Elaeis Guineensis Palmöl (CEGESOFT^{®} GPO), Passiflora Incarnata Saatöl (CEGESOFT^{®} PFO), Olus Öl (CEGESOFT^{®} PS 6), Ethylhexylstearat (CETIOL^{®} 868), Hexyllaurat (CETIOL^{®} A), C12-15 Alkylbenzoat (CETIOL^{®} AB), Dibutyladipat (CETIOL^{®} B), Coco-Caprylat (CETIOL^{®} C5), Coco-Caprylat/Caprat (CETIOL^{®} C 5C), Dicaprylylcarbonat (CETIOL^{®} CC), Ethylhexylcocoat (und) Cocos Nucifera Öl (CETIOL^{®} COCO), PPG-15 Stearylether (CETIOL^{®} E), Oleylerucat (CETIOL^{®} J 600), Hexyldecanol (und) Hexyldecyl Laurat (CETIOL^{®} PGL), Caprylyl-Caprylat/Caprat (CETIOL^{®} RLF), Propylheptylcaprylat (CETIOL^{®} SENSOFT), Cetearylisononanoat (CETIOL^{®} SN), Decyloleat (CETIOL^{®} V), Octyldodecanol (EUTANOL^{®} G), Hexyldecanol (EUTANOL^{®} G16), Hexyldecylstearat (EUTANOL^{®} G16S), Isopropylmyristat, Isopropylpalmitat, Cetearylethylhexanoat (LUVITOL^{®} EHO), Capryl/Caprin-Triglycerid (MYRITOL^{®} 312), Capryl/Caprin-Triglycerid (MYRITOL^{®} 318), Cocoglycerid (MYRITOL^{®} 331).

Ein ganz bevorzugtes Öl ist Coco-Caprylat/Caprat.

Typische Beispiele für Fette sind Glyceride, d.h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oderstearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Die Emulgatorkombination in der erfindungsgemäßen texturierten Zusammensetzung beträgt bevorzugter Weise 3,5 bis 5,5 Gew.-%.

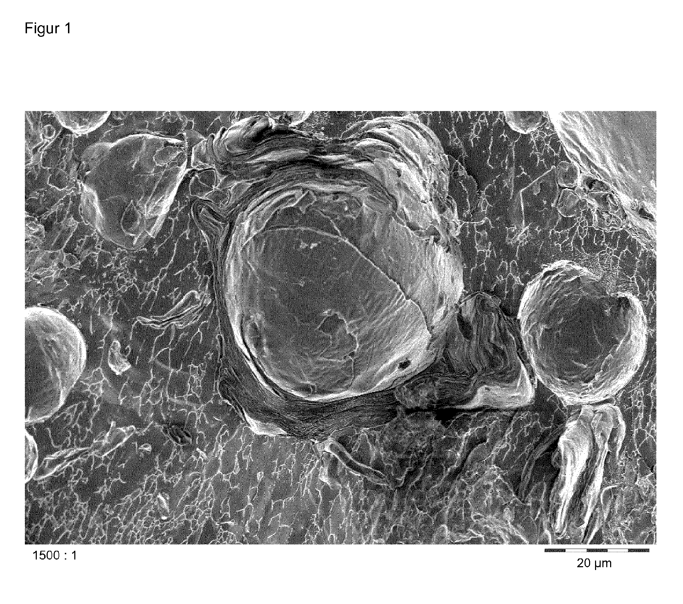

Die erfindungsgemäße texturierte Zusammensetzung weist eine Crushed Ice-Struktur auf, die in einem Öl-in-Wasser-System durch ein lamellares Gelnetzwerk stabilisiert ist. Diese besondere Textur wurde mittels Rasterelektronenmikroskopie auf molekularer Ebene untersucht. Dazu wurde die Probe im Rasterelektronenmikroskop gesichtet und mittels Sekundärelektronen bei 2kV im eingefrorenen Zustand an exemplarischen Stellen topographisch abgebildet. Die Form, Größe und Morphologie dieser Öl-in-Wasser-Emulsionen kann anhand der Figuren 1 bis 4 beurteilt werden.

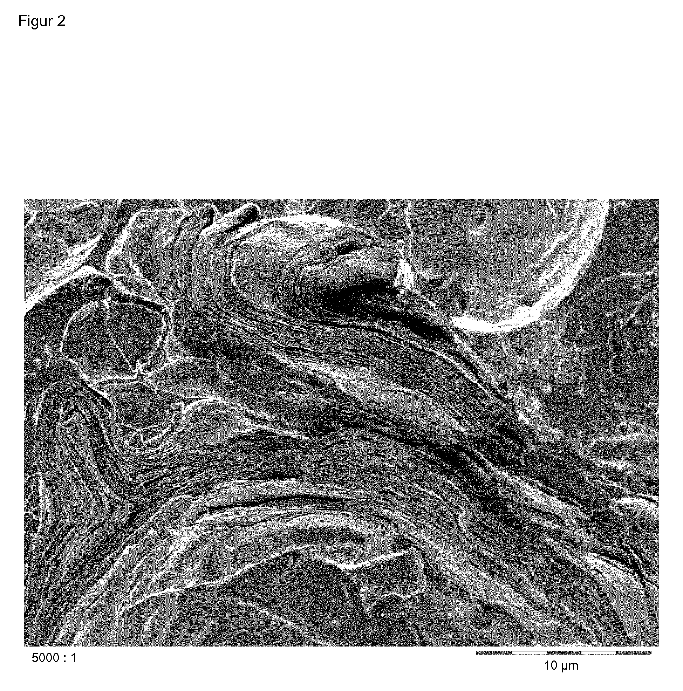

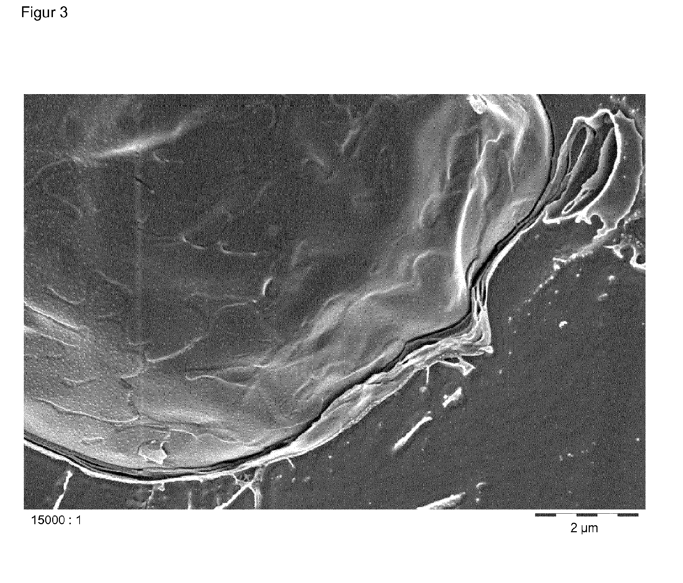

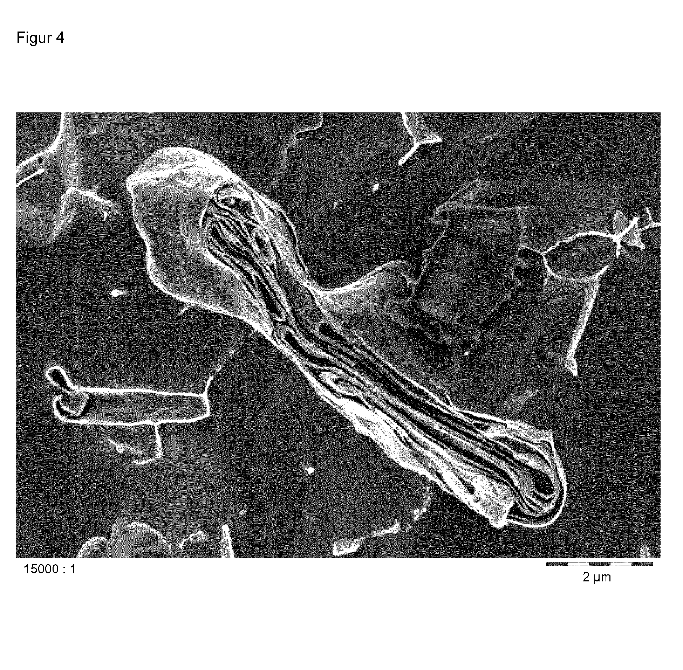

Figur 1 zeigt in 1500-facher Vergrößerung die Öltröpfchen in der Emulsion in verschiedenen Größen mit bis zu ca. 100 µm Durchmesser. Lamellare Schichten sind sowohl um die Öltröpfchen herum als auch in der Wasserphase zu erkennen. Die Figur 3 zeigt in 1500-facher Vergrößerung ein Öltröpfchen mit einem Kranz von lamellaren Schichten mit einigen µm Dicke. Die Figur 2 lässt erkennen, dass sich ebenfalls lamellare Schichten in der Wasserphase ausgebildet haben. Die 15000-fache Vergrößerung einer lamellaren Struktur in Figur 4 zeigt den lamellaren Aufbau einer Schichtstruktur in der Wasserphase.

Die erfindungsgemäße texturierte Zusammensetzung wird durch einfaches Rühren der Bestandteile ohne Homogenisierung hergestellt. Auf diese Weise kann sich die lamellare Netzwerkstruktur ausbilden. Dazu werden die Bestandteile nach dem Erhitzen auf 75 bis 85°C vereint und nach der Phasenvereinigung unter langsamem Rühren mit einer Umdrehungszahl < 600 U/min bis zum Erreichen der Raumtemperatur gerührt. Bevorzugt beträgt die Umdrehungszahl bei der Rührung ≤ 300 U/min. Die erhaltene Zusammensetzung ist lagerstabil und erhält ihre Textur über einen Zeitraum von bis zu 3 Jahren.

Überraschenderweise können durch Veränderung der Mengen bzw. Konzentration der in der texturierten Zusammensetzung der Erfindung enthaltenen Bestandteile verschiedene weitere, davon unterschiedliche Texturen hergestellt werden. So kann durch das Weglassen der Ölphase ein texturiertes Gel dargestellt werden.

Ein weiterer Gegenstand der vorliegenden Erfindung ist eine texturierte Zusammensetzung, die in einem wässrigen System ein Hydrogel bildet und die Bestandteile aufweist:
- Eine Emulgatorkombination aus
   0,05 bis 3,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- bis 100 Gew.-% Wasser.

Es bildet sich ein Gel mit darin enthaltenen festen körnigen Strukturen, gleich einem Peeling-Gel. Das Gel wird, wie die obige Zusammensetzung, ohne Homogenisierung nur durch moderates Rühren hergestellt. Es ist über bis zu 3 Jahren lagerstabil. Auch bei höheren Temperaturen (>40°C) oder Wechseltemperaturen (-5°C bis 40°C) sowie Gefriertemperaturen wird eine Lagerstabilität bis zu 6 Monaten erreicht.

Eine weitere texturierte Zusammensetzung mit einer Mousse-Struktur, die schaumförmig, souffléeartig, gashaltig und schäumbar ist, lässt sich darstellen, indem das Verhältnis von anionischem Emulgator zu Fettalkohol zu Gunsten des anionischen Emulgators verändert wird, das heißt, dass mehr anionischer Emulgator als Fettalkohol eingesetzt wird.

Somit ist ein weiterer Gegenstand der vorliegenden Erfindung eine texturierte Zusammensetzung, die in einem Öl-in-Wasser-System eine schaumartige Struktur bildet und die Bestandteile aufweist:
- Eine Emulgatorkombination aus
   0,05 bis 3,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- 3,00 bis 20,0 Gew.-% mindestens ein Öl und/oder Wachs, und
- bis 100 Gew.-% Wasser,
wobei die Emulgatorkombination 3,5 bis 5,5 Gew.-% beträgt und das Verhältnis von anionischem Emulgator zu Fettalkohol im Bereich von 6:1 bis 3:2 liegt.

Die in der weichen Textur eingeschlossenen Hohlräume sind mit Luft gefüllt und verleihen der Zusammensetzung eine Textur eine besondere Leichtigkeit bei der Applikation.

Diese schaumige Zusammensetzung ist über einen Zeitraum von bis zu 3 Jahren lagerstabil.

Eine weitere Textur, die sich als Creme-Emulsion darstellt, lässt sich dadurch herstellen, indem das Verhältnis von anionischen Emulgator zu Fettalkohol gleich 1 gesetzt wird und die Emulgatorkonzentration in der Emulsion stärker variabel gestaltet ist.

Schließlich umfasst die vorliegende Erfindung auch eine Creme-artige texturierte Zusammensetzung, die in einem Öl-in-Wasser-System eine feindisperse Tröpfchenverteilung mit lamellaren Strukturen bildet und die Bestandteile aufweist:
- Eine Emulgatorkombination aus
   0,05 bis 5,00 Gew.- mindestens einem anionischen Emulgator und
   0,15 bis 9,00 Gew.-% mindestens einem Fettalkohol,
- 0,01 bis 5,00 Gew.-% mindestens ein Hydrokolloid und
- 0,10 bis 20,0 Gew.-% mindestens ein Öl und/oder Wachs, und
- bis 100 Gew.-% Wasser,
wobei die Emulgatorkombination 2,00 bis 8,00 Gew.-% beträgt und das Verhältnis von anionischem Emulgator zu Fettalkohol 1:1 beträgt.

Diese Emulsion ist über einen Zeitraum von bis zu 3 Jahren lagerstabil.

Die bevorzugten Mengenbereiche für die einzelnen Komponenten der genannten drei Zusammensetzungen bzw. Texturen sind die gleichen wie bei der Zusammensetzung mit Crushed-Ice-Textur. Des Weiteren gelten für diese Zusammensetzungen auch die gleichen Komponenten wie für die Zusammensetzung mit Crushed-Ice-Textur.

Die Zusammensetzungen mit den schaumartigen und Creme-artigen Texturen werden in üblicher Weise durch moderates Rühren, wie oben beschrieben und gegebenenfalls anschließender Homogenisierung hergestellt.

Dazu werden die Bestandteile nach dem Erhitzen auf 75 bis 85°C vereint, unter langsamen Rühren mit einer Umdrehungszahl kleiner 600 U/min, bevorzugt kleiner/gleich 300 U/min, bis zum Erreichen einer Temperatur von 55 bis 75°C gerührt, wonach anschließend homogenisiert wird und dann wird mit einer Umdrehungszahl kleiner 600 U/min, bevorzugt kleiner/gleich 300 U/min, bis zum Erreichen der Raumtemperatur gerührt wird.

Die Homogenisierung wird mit einer geeigneten Apparatur, zum Beispiel mit einem Rotor-stator oder Ultra-Turrax, unter Bildung einer Emulsion homogenisiert.

Die erfindungsgemäßen texturierten Zusammensetzungen sind hervorragend auf dem Gebiet der Kosmetik einzusetzen. Daher betrifft die vorliegende Erfindung ebenfalls texturierte kosmetische Zusammensetzungen, die in den oben beschriebenen Texturen vorliegen. Diese kosmetischen Zusammensetzungen enthalten weiterhin kosmetisch aktive Bestandteile aus der Gruppe Pigmente, Pflanzenextrakte, Peptide, Proteine, marines Atelocollagen, Phytoceramide, Phytosterole, Polyphenole, Polyole, Harnstoff, Hyaluronsäure, Zucker und Zuckerderivate, Natrium-PCA, Vitamine, UV-Lichtschutzfilter, Antioxidantien, biogene Wirkstoffe, Selbstbräuner, Konservierungsmittel, Parfümöle, Pflanzenöle, Antitranspirantien, Esteraseinhibitoren, Bakterizide und Mischungen davon.

Die erfindungsgemäßen Zusammensetzungen können in Form von Cremes, Milch, Lotionen Gelen, Stiften, Conditioner, Sprays, Serum, Aerosolschaum, Pumpschaum, Pasten oder Wachse vorliegen.

Beispiele für diese kosmetisch aktiven Bestandteile sind nachfolgend beschrieben.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Trüsopropylcitrat, Tributylcitrat und insbesondere Triethylcitrat (Hydagen® CAT, BASF AG, Düsseldorf). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z.B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

### UV-Lichtschutzfilter

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher;
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)-[2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl XL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4`-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
➢ 2,2(-Methylen-bis(6-(2H-benzotriazol-2-yl)-4-(1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4`methoxyphenyl)propan-1,3-dion;
➢ Ketotricyclo(5.2.1.0)decan-Derivate,
➢ Dimethicodiethylbenzalmalonate (Parsol SLX).

### Als wasserlösliche UV - Filter kommen in Frage:

➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4`-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

### Pigmente

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. - Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, - Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Nichtionische Emulgatoren

Nichtionische Emulgatoren können ebenfalls in Kombination mit den anionischen Emulgatoren vorhanden sein. Beispiele für diese nichtionischen Emulgatoren sind Fettalkoholpolyglycolether (Eumulgin S 2, Eumulgin S 21, Eumulgin B1, Eumulgin B2, Cremophor A 25, Eumulgin B3, BASF und Emulgatorcompounds, wie Emulgade 1000 NI, Lanette WAX AO, Emulgade SE PF, Emulgade NLB, Alkylphenolpolyglycolether, Fettsäurepolyglycolester, wie Emulgade 165 Cremophor GS 32 , Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate, insbesondere pflanzliche Produkte auf Weizenbasis, Polyolfettsäureester, Zuckerester (wie Emulgade PL 68/50, Emulgade Sucro von der BASF), Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Der Anteil der nichtionischen Emulgatoren liegt üblicherweise bei etwa 0,01 bis 10,00 vorzugsweise 0,05 bis 5,00 und insbesondere 0,05 bis 3,00 Gew.-%.

### Verdickungsmittel

Als Verdickungsmittel eignen sich beispielsweise Aerosil-Typen (hydrophile Kieselsäuren), Schichtsilikate, wie Magnesium-Aluminium-Silikat, Polyvinylalkohol, Polyvinylpyrrolidon und Bentonite wie z. B. Bentone^{®} Gel VS-5PC (Rheox), Taurate und deren Derivate, Polyurethane, Polyacrylamide, PVM/MA-Copolymere und Mischungen ausgewählt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die Erfindung wird nun nachfolgend anhand der Beispiele erläutert.

### 5. Beispiele

### Beispiel 1 bis 3: Zusammensetzungen mit der Textur "Crushed Ice"

| | | | 1 | 2 | 3 | |
|---|---|---|---|---|---|---|
| Phase | Bestandteile | INCI | % | % | % | Funktion |
| I | Cutina GMS-SE | Glycerylstearat SE | 0,50 | | | Emulgator |
| | Eumulgin SG | Natriumstearoyl-Glutamate | | 0,05 | | Emulgator |
| | Eumulgin Prisma | Dinatrium-Cetearyl-Sulfosuccinate | | | 0,05 | Emulgator |
| | Cetiol LC | Coco-Caprylat/Caprat | 15,00 | 15,00 | 15,00 | Öl |
| | Lanette 16 | Cetylalkohol | 0,90 | 0,90 | 0,90 | Fettalkohol |
| | Lanette 18 | Stearylalkohol | 2,10 | 2,10 | 2,10 | Fettalkohol |
| II | Rheocare C Plus | Carbomer | 0,20 | 0,20 | 0,20 | Rheologiemodifizierungsmittel |
| | Wasser demin. | | 80,45 | 80,90 | 80,90 | |
| | Hydantoin DMDMH | Dimethyloldimethylhydantoin | 0,70 | 0,70 | 0,70 | Konservierungsmittel |
| III | KOH 20% | Kaliumhydroxid | 0,15 | 0,15 | 0,15 | Neutralisationsmittel |
| IV | Parfum | | q.s | q.s | q.s | |
| | Soll pH 6,5 | | 6,40 | 6,40 | 6,40 | |

Die Zusammensetzungen werden wie folgt hergestellt: Erhitzen der Phasen I und II auf 75 bis 85°C. Vereinigung der Phasen I und II unter langsamen Rühren mit einer Umdrehungszahl kleiner 400 U/min bis zum Erreichen einer Temperatur von 55-60°C. Zugabe der Phase III und Rühren mit einer Umdrehungszahl bei 400 U/min, bis zum Erreichen der Raumtemperatur unter Zugabe des Parfums.

### Beispiel 4: Zusammensetzung mit Textur "Peeling-Gel"

| Phase | Bestandteile | INCI | % | Funktion |
|---|---|---|---|---|
| I | Cutina GMS-SE | Glycerylstearat SE | 1,00 | Emulgator |
| | Lanette 0 | Cetearylalkohol | 3,00 | Fettalkohol |
| II | Rheocare C Plus | Carbomer | 0,20 | Rheologiemodifizierungsmittel |
| | Wasser, demin. | | 94,85 | |
| | Hydantoin DMDMH | Dimethyloldimethyl-hydantoin | 0,70 | Konservierungsmittel |
| III | KOH 20% | Kaliumhydroxid | 0.15 | Neutralisationsmittel |
| | Parfum | | q.s. | |
| | Soll pH 6,5 | | 6,10 | |

Herstellung der Zusammensetzung: Erhitzen der Phasen I und II auf 75 bis 85°C. Vereinigung der Phasen I und II unter langsamen Rühren mit einer Umdrehungszahl kleiner 300 U/min bis zum Erreichen einer Temperatur von 55-60°C. Zugabe der Phase III und Rühren mit einer Umdrehungszahl bei 300 U/min bis zum Erreichen der Raumtemperatur unter Zugabe des Parfums.

### Beispiel 5: Zusammensetzung mit Textur "Creme"

| Phase | Bestandteile | INCI | % | Funktion |
|---|---|---|---|---|
| I | Cutina GMS-SE | Glycerylstearat SE | 3,00 | Emulgator |
| | Cetiol LC | Coco-Caprylat/Caprat | 15,00 | Öl |
| | Lanette 16 | Cetylalkohol | 0,90 | Fettalkohol |
| | Lanette 18 | Stearylalkohol | 2,10 | Fettalkohol |
| II | Rheocare C Plus | Carbomer | 0,20 | Rheologiemodifizierungsmittel |
| | Wasser, demin. | | 77,95 | |
| | Hydantoin DMDMH | Dimethyloldimethylhydantoin | 0,70 | Konservierungsmittel |
| III | KOH 20% | Kaliumhydroxid | 0,15 | Neutralisationsmittel |
| | Soll pH 6,5 | | 6,60 | |

Die Zusammensetzung wird wie folgt hergestellt: Erhitzen der Phasen I und II auf 75 bis 85°C. Vereinigung der Phasen I und II unter langsamen Rühren mit einer Umdrehungszahl kleiner 300 U/min bis zum Erreichen einer Temperatur von 55-60°C. Zugabe der Phase III und optional Homogenisierung mit einem Rotor-Stator für > 5 Minuten. Danach wird mit einer Umdrehungszahl kleiner 300 U/min bis zum Erreichen der Raumtemperatur gerührt.

### Beispiele 6 und 7: Zusammensetzungen mit Textur "Mousse"

| | | 6 | 7 | |
|---|---|---|---|---|
| Bestandteile | INCI | % | % | Funktion |
| Cutina GMS-SE | Glycerylstearat SE | 3,00 | 3,00 | Emulgator |
| Cetiol LC | Coco-Caprylat/Caprat | 15,00 | 15,00 | Öl |
| Lanette O | Cetearylalkohol | 0,50 | 2,00 | Fettalkohol |
| Rheocare C Plus | Carbomer | 0,20 | 0,20 | Rheologiemodifizierungsmittel |
| Wasser, demin. | | 80,35 | 78,85 | |
| Hydantoin DMDMH | Dimethyloldimethylhydantoin | 0,80 | 0,80 | Konservierungsmittel |
| KOH 20% | Kaliumhydroxid | 0,15 | 0,15 | Neutralisationsmittel |
| Soll pH 6,5 | | 6,20 | 6,60 | |

Herstellung der Zusammensetzung: Erhitzen der Phasen I und II auf 75 bis 85°C. Vereinigung der Phasen I und II unter langsamen Rühren mit einer Umdrehungszahl kleiner 300 U/min bis zum Erreichen einer Temperatur von 55-60°C. Zugabe der Phase III und optional starke Homogenisierung (Rotor-Stator für > 5 min). Danach wird mit einer Umdrehungszahl kleiner 300 U/min bis zum Erreichen der Raumtemperatur gerührt.

## Patentansprüche

1. Texturierte Zusammensetzung, die in einem Öl-in-Wasser-System ein lamellares Gelnetzwerk bildet und die Bestandteile aufweist:
- Eine Emulgatorkombination aus
0,05 bis 3,00 Gew.- mindestens einem anionischen Emulgator und
0,15 bis 9,00 Gew.-% mindestens einem Fettalkohol,
- 0,01 bis 5,00 Gew.-% mindestens ein Hydrokolloid und
- 3,00 bis 20,0 Gew.-% mindestens ein Öl und/oder Wachs, und
- bis 100 Gew.-% Wasser,
wobei das Verhältnis von anionischem Emulgator zu Fettalkohol im Bereich von 1:6 bis 2:3 liegt.

2. Zusammensetzung nach Anspruch 1, **dadurch gekennzeichnet, dass** der mindestens eine Fettalkohol in einer Konzentration von 0,5 bis 6,00 Gew.-% enthalten ist.

3. Zusammensetzung nach Anspruch 1 und/oder 2, **dadurch gekennzeichnet, dass** das mindestens eine Hydrokolloid in einer Konzentration von 0,1 bis 1,00 Gew.-% enthalten ist.

4. Zusammensetzung nach einem der Ansprüche 1 bis 3, **dadurch gekennzeichnet, dass** das mindestens eine Öl in einer Konzentration von 5,00 bis 15,00 Gew.-% enthalten ist.

5. Zusammensetzung nach einem der Ansprüche 1 bis 4, **dadurch gekennzeichnet, dass** der anionische Emulgator mindestens einen Emulgator aus der Gruppe Salze der Fettsäuren, Salze der Fettsäuren in Kombination mit Mono-/Diglyceriden der Fettsäuren, Salze der Stearoylmilchsäure, Salze der Stearoylglutaminsäure bzw. Alkylglutamate, Alkylphosphate, Alkylsulfate, Alkylsarkosinate, Salze der Alkylsulfobernsteinsäure und Salze der Zitronensäureester umfasst.

6. Zusammensetzung nach einem der Ansprüche 1 bis 5, **dadurch gekennzeichnet, dass** der Fettalkohol mindestens einen Fettalkohol der Formel (I)
R-OH (I)
umfasst, worin R ein aliphatischer, linearer oder verzweigter Alkylrest mit 6 bis 22 C- Atomen, bevorzugt 14 bis 20 C-Atomen, mit 0 und/oder 1, 2 oder 3 Doppelbindungen bedeutet.

7. Zusammensetzung nach einem der Ansprüche 1 bis 6, **dadurch gekennzeichnet, dass** das Hydrokolloid aus der Gruppe Acrylsäure und Acrylsäurederivate, Kohlenhydrate, wie Cellulose und natürliche Gummi und deren Derivate, Taurate und deren Derivate, Polyurethane, Polyacrylamide, PVM/MA-Copolymere, VP-Copolymer, VA-Copolymere und Mischungen daraus gewählt ist.

8. Zusammensetzung nach einem der Ansprüche 1 bis 7, **dadurch gekennzeichnet, dass** das Öl oder Wachs aus der Gruppe Fettsäureester, Kohlenwasserstoffe, Guerbetalkohole, Tri- oder Partialglyceride, Mono-/Dialkylether, Mono-/Dialkylcarbonate, öllösliche UV-Filter, Fettalkoholether, mikrokristalline Wachse, Mineralöl, Silikonöl, natürliche pflanzliche Öle und deren Mischungen gewählt ist.

9. Zusammensetzung nach einem der Ansprüche 1 bis 8, **dadurch gekennzeichnet, dass** die Emulgatorkombination 3,5 bis 5,5 Gew.-% beträgt.

10. Texturierte Zusammensetzung, die in einem wässrigen System ein Hydrogel bildet und die Bestandteile aufweist:
- Eine Emulgatorkombination aus
0,05 bis 3,00 Gew.- mindestens einem anionischen Emulgator und
0,15 bis 9,00 Gew.-% mindestens einem Fettalkohol,
- 0,01 bis 5,00 Gew.-% mindestens ein Hydrokolloid und
- bis 100 Gew.-% Wasser,
wobei das Verhältnis von anionischem Emulgator zu Fettalkohol im Bereich von 1:3 beträgt.

11. Texturierte Zusammensetzung, die in einem Öl-in-Wasser-System eine schaumartige Struktur bildet und die Bestandteile aufweist:
- Eine Emulgatorkombination aus
0,05 bis 3,00 Gew.- mindestens einem anionischen Emulgator und
0,15 bis 9,00 Gew.-% mindestens einem Fettalkohol,
- 0,01 bis 5,00 Gew.-% mindestens ein Hydrokolloid und
- 3,00 bis 20,0 Gew.-% mindestens ein Öl und/oder Wachs, und
- bis 100 Gew.-% Wasser,
wobei die Emulgatorkombination 3,5 bis 5,5 Gew.-% beträgt und das Verhältnis von anionischem Emulgator zu Fettalkohol im Bereich von 6:1 bis 3:2 liegt.

12. Texturierte Zusammensetzung, die in einem Öl-in-Wasser-System eine feindisperse Tröpfchenverteilung mit lamellaren Strukturen bildet und die Bestandteile aufweist:
- Eine Emulgatorkombination aus
0,05 bis 5,00 Gew.- mindestens einem anionischen Emulgator und
0,15 bis 9,00 Gew.-% mindestens einem Fettalkohol,
- 0,01 bis 5,00 Gew.-% mindestens ein Hydrokolloid und
- 3,00 bis 20,0 Gew.-% mindestens ein Öl und/oder Wachs, und
- bis 100 Gew.-% Wasser,
wobei die Emulgatorkombination 2,00 bis 8,00 Gew.-% beträgt und das Verhältnis von anionischem Emulgator zu Fettalkohol 1:1 beträgt.

13. Verfahren zur Herstellung einer texturierten Zusammensetzung nach einem der Ansprüche 1 bis 12, wobei die Bestandteile nach dem Erhitzen auf 75 bis 85°C vereint werden und nach Phasenvereinigung unter langsamen Rühren mit einer Umdrehungszahl kleiner 600 U/min, bevorzugt kleiner/gleich 300 U/min, bis zum Erreichen der Raumtemperatur gerührt werden.

14. Verfahren zur Herstellung einer texturierten Zusammensetzung nach Anspruch 11 oder 12, wobei die Bestandteile nach dem Erhitzen auf 75 bis 85°C vereint werden, unter langsamen Rühren mit einer Umdrehungszahl kleiner 600 U/min, bevorzugt kleiner/gleich 300 U/min, bis zum Erreichen einer Temperatur von 55 bis 75°C gerührt werden, anschließend homogenisiert wird und dann mit einer Umdrehungszahl kleiner 600 U/min, bevorzugt kleiner/gleich 300 U/min, bis zum Erreichen der Raumtemperatur gerührt wird.

15. Texturierte kosmetische Zusammensetzung nach einem der Ansprüche 1 bis 14, die weiterhin kosmetisch aktive Bestandteile aus der Gruppe Pigmente, Pflanzenextrakte, Peptide, Proteine, marines Atelocollagen, Phytoceramide, Phytosterole, Polyphenole, Polyole, Harnstoff, Hyaluronsäure, Zucker und Zuckerderivate, Natrium-PCA, Vitamine, UV-Lichtschutzfilter, Antioxidantien, biogene Wirkstoffe, Selbstbräuner, Konservierungsmittel, Parfümöle, Pflanzenöle, Antitranspirantien, Esteraseinhibitoren, Bakterizide und Mischungen davon enthält.
